# EUROPEAN PATENT APPLICATION

(11) **EP 4 477 739 A1**
(43) Date of publication of application: **18.12.2024**
(21) Application number: 23749899.3
(22) Date of filing: 07.02.2023
(51) Int. Cl.: C12N 1/21, C12N 15/31, C12N 15/55, C12N 15/62, C12N 15/74, C12P 19/14

(54) **CELLULOSE-SYNTHETIZING MICROBE TRANSFECTANT AND USE THEREOF**

(30) Priority: 07.02.2022 JP 2022017356
(71) Applicant: Sumitomo Chemical Company, Limited, Tokyo 103-6020 (JP); NATIONAL UNIVERSITY CORPORATION KYOTO INSTITUTE OF TECHNOLOGY, Kyoto-shi, Kyoto 606-8585 (JP)
(72) Inventor: ASO, Yuji, Kyoto-shi, Kyoto 606-8585 (JP)
(74) Representative: Vossius & Partner Patentanwälte Rechtsanwälte mbB
(86) International application number: PCT/JP2023/003994
(87) International publication number: WO 2023/149583

(57) **Abstract**

An object of the present invention is to realize a transformant of a cellulose-synthesizing microorganism that is capable of producing glucose or cellobiose from a carbon source such as methanol. A transformant in accordance with an embodiment of the present invention attains the above object by being obtained by introducing a cellulase gene and a cell surface protein gene into a cellulose-synthesizing microorganism.

## Description

### Technical Field

The present invention relates to a transformant of a cellulose-synthesizing microorganism and use thereof.

### Background Art

Glucose is an energy source that is important for living organisms, and animals take in glucose mainly produced from CO₂ through photosynthesis of plants or the like. Since glucose is useful as food, feed, a nutrient source for a microorganism, and a raw material for a chemical product, various methods have been studied as a method for more efficiently producing glucose.

For example, Patent Literature 1 discloses a yeast capable of producing glucose from cellulose and a method for producing glucose from cellulose with use of the yeast. In addition, Non-Patent Literature 1 discloses a technique for producing glucose in a bacterial cell with use of cyanobacteria, and exporting glucose produced by a transporter to the outside of the bacterial cell.

### Citation List

### [Patent Literature]

[Patent Literature 1]
International Publication No. WO 2009/128305

### [Non-patent Literature]

[Non-patent Literature 1]
APPLIED AND ENVIRONMENTAL MICROBIOLOGY, June 2010, p.3462-3466

### Summary of Invention

### Technical Problem

However, a method of biochemically synthesizing glucose from a carbon source such as methanol without relying on photosynthesis has not been known. Further, a method of producing cellobiose, which is a disaccharide that is composed of two glucose molecules bound to each other, through biosynthesis without relying on photosynthesis has not been known either.

The present invention has been attained in view of the above problems, and an object of the present invention is to realize a transformant of a cellulose-synthesizing microorganism that is capable of producing glucose or cellobiose from a carbon source such as methanol.

### Solution to Problem

As a result of conducting diligent studies in order to solve the above problems, the inventor of the present invention and others have found that it is possible to produce glucose or cellobiose from a carbon source such as methanol by using a transformant having a cellulase gene and a gene that expresses the cellulase on a cell surface introduced thereinto or a transformant of a bacterium belonging to the family *Methylobacteriaceae* that has become capable of expressing cellulase on a bacterial cell surface by introduction of an oprI gene.

That is, the present invention includes the following features:
A transformant obtained by introducing a cellulase gene and a cell surface protein gene into a cellulose-synthesizing microorganism.

A transformant obtained by introducing an oprI gene into a bacterium belonging to the family *Methylobacteriaceae.*

### Advantageous Effects of Invention

According to an aspect of the present invention, it is possible to provide a transformant of a cellulose-synthesizing microorganism that is capable of producing glucose or cellobiose from a carbon source such as methanol.

### Brief Description of Drawings

Fig. 1 is a schematic diagram of a transformant in accordance with Example of the present invention.
Fig. 2 is a graph showing a fluorescence intensity of a cellulose-synthesizing bacterium in accordance with Example of the present invention.
Fig. 3 is a schematic diagram of a transformant in accordance with Example of the present invention.
Fig. 4 is a graph showing a methanol consumption, a dried bacterial cell weight, and a glucose yield of a wild strain of a cellulose-synthesizing bacterium in accordance with Example of the present invention.
Fig. 5 is a graph showing a methanol consumption, a dried bacterial cell weight, and a glucose yield of a transformant of a cellulose-synthesizing bacterium in accordance with Example of the present invention.
Fig. 6 is a graph showing cellulase activity of a cellulose-synthesizing bacterium in accordance with Example of the present invention.
Fig. 7 is a graph showing changes over time of cellulase activity of a cellulose-synthesizing bacterium in accordance with Example of the present invention.
Fig. 8 is a graph showing culture solution turbidities and glucose yields of a wild strain of a cellulose-synthesizing bacterium in accordance with Example of the present invention in a case where varying substrates were used.
Fig. 9 is a graph showing culture solution turbidities and glucose yields of a transformant of a cellulose-synthesizing bacterium in accordance with Example of the present invention in a case where varying substrates were used.
Fig. 10 is a schematic diagram of a transformant in accordance with Example of the present invention.
Fig. 11 is a graph showing a cellobiose yield of a transformant of a cellulose-synthesizing bacterium in accordance with Example of the present invention.
Fig. 12 is an HPLC chromatogram of a transformant of a cellulose-synthesizing bacterium in accordance with Example of the present invention.

### Description of Embodiments

The following description will discuss embodiments of the present invention. The present invention is not, however, limited to these embodiments. Any numerical range expressed as "A to B" herein means "not less than A and not more than B" unless otherwise stated.

### [1. Transformant]

A transformant in accordance with an embodiment of the present invention (hereinafter also referred to as present transformant) is obtained by introducing a cellulase gene and a cell surface protein gene into a cellulose-synthesizing microorganism.

The present transformant contains a cellulase gene and a cell surface protein gene and thus enables cellulase to be expressed on a cell surface thereof. Any technique for causing a cellulose-synthesizing microorganism to express cellulase on a cell surface thereof did not conventionally exist. Further, according to the present transformant, it is possible to produce glucose or cellobiose from cellulose without adding cellulase from the outside. In addition, the present transformant is capable of producing glucose or cellobiose with high productivity (specific productivity) per cell and at higher yield.

The cellulose-synthesizing microorganism can produce cellulose from a carbon source via a serine circuit or the like. Therefore, according to the present invention, by using, as the carbon source, for example, CO₂-derived methanol that is produced for commercial use, glucose or cellobiose can be produced from CO₂ without relying on photosynthesis.

Further, the above-described configuration does not rely on photosynthesis and thus enables glucose or cellobiose to be obtained even in non-arable land. The obtained glucose or cellobiose can be utilized as food, feed, a nutrient source for microorganism culture, and a raw material for a chemical product, and thus enables biochemical synthesis of various substances. This makes it possible to contribute to achievement and realization of, for example, Goal 2 "Ensure a sustainable food production system" of Sustainable Development Goals (SDGs).

The introduction of the cellulase gene and the cell surface protein gene into the cellulose-synthesizing microorganism can be carried out in accordance with a known method. Specifically, the introduction is achieved by, for example, introducing a plasmid vector that has a sequence of a cellulase gene and a sequence which encodes a protein that expresses the cellulase gene on a cell surface into the cellulose-synthesizing microorganism by use of an electroporation method or the like.

In the present specification, the "cellulose-synthesizing microorganism" means bacteria, archaeobacteria, and fungi which are capable of synthesizing cellulose. Among these, the cellulose-synthesizing microorganism is preferably a bacterium, that is, a cellulose-synthesizing bacterium. Thus, the "productivity per cell" can also be said to be, more specifically, productivity per bacterial cell.

In the present specification, the cellulose-synthesizing bacterium is not particularly limited, provided that it is a bacterium capable of synthesizing cellulose from a carbon source, and may be a bacterium belonging to the family *Methylobacteriaceae* capable of synthesizing cellulose or a bacterium other than the bacterium belonging to the family *Methylobacteriaceae.*

The bacterium belonging to the family *Methylobacteriaceae* may be a bacterium belonging to the genus *Methylorubrum* capable of synthesizing cellulose. Examples of the bacterium belonging to the genus *Methylorubrum* capable of synthesizing cellulose include *Methylorubrum extorquens, Methylorubrum podarium*, *Methylorubrum populi, Methylorubrum rhodesianum*, *Methylorubrum salsuginis*, *Methylorubrum zatmani*, and the like. Among these bacteria, the bacterium belonging to the genus *Methylorubrum* capable of synthesizing cellulose is preferably *Methylorubrum extorquens,* and more preferably *Methylorubrum extorquens* AM1. Since the *Methylorubrum extorquens* is a bacterium that does not utilize glucose, produced glucose is less likely to be consumed by the *Methylorubrum extorquens.* Therefore, a glucose yield is improved. A bacterium capable of synthesizing cellulose from a carbon source other than the bacteria belonging to the family *Methylobacteriaceae* is preferably at least one bacterium selected from *Agrobacterium tumefaciens*, *Rhizobium leguminosarum*, *Salmonella enterica*, *Escherichia coli, Komagataeibacter medellinensis,* and *Komagataeibacter hansenii.* Since a gene recombination method for these cellulose-synthesizing bacteria has been established, transformants of these cellulose-synthesizing bacteria are easily produced.

The cell surface protein gene in the present transformant means a gene that expresses cellulase on a cell surface. The cell surface protein gene may be a bacterial cell surface protein. Examples of the bacterial cell surface protein gene include a lipoprotein gene, an S-layer protein gene, a pilin gene, a flagellin gene, an outer membrane protein gene, and the like. Examples of the outer membrane protein gene include an oprI gene, a lamB gene, an ompT gene, a lpp-ompA gene, and the like. Among these genes, the outer membrane protein gene is preferably an oprI gene. The oprI gene is a gene that encodes a major outer membrane lipoprotein (oprI), which is a bacterial cell outer membrane protein derived from *Pseudomonas aeruginosa.*

In an embodiment of the present invention, the oprI gene is preferably any DNA selected from the group consisting of the following (1) to (6):
(1) DNA consisting of a base sequence of SEQ ID NO: 1;
(2) DNA encoding a polypeptide that has not less than 90% homology to the base sequence of SEQ ID NO: 1 and that is a cell surface protein;
(3) DNA (i) hybridizing, under a stringent condition, with a gene consisting of a base sequence complementary to the base sequence of SEQ ID NO: 1 and (ii) encoding a polypeptide that is a cell surface protein;
(4) DNA encoding a polypeptide consisting of an amino acid sequence of SEQ ID NO: 2;
(5) DNA encoding a polypeptide that has not less than 90% identity to the amino acid sequence of SEQ ID NO: 2 and that is a cell surface protein; and
(6) DNA encoding a polypeptide (i) consisting of an amino acid sequence in which at least one amino acid is deleted, substituted, inserted, or added in the amino acid sequence of SEQ ID NO: 2 and (ii) being a cell surface protein.

The SEQ ID NO: 1 corresponds to the full-length base sequence of the oprI gene. Further, the SEQ ID NO: 2 corresponds to the full-length amino acid sequence encoded by the oprI gene.

With regard to (2) above, the homology to the base sequence is preferably not less than 92%, more preferably not less than 95%, and even more preferably not less than 99%. Note that the homology to a base sequence of a gene can be determined by a gene analysis program such as BLAST (http://blast.genome.ad.jp) or FASTA (http: // fasta.genome.ad.jp/SIT/ FASTA.html).

With regard to (3) above, the term "stringent condition" indicates, in general, a condition under which a double-stranded polynucleotide that is specific to a base sequence is formed, and a double-stranded polynucleotide that is not specific to a base sequence is not formed. In other words, the "stringent condition" can be expressed as a condition under which hybridization is carried out at a temperature in a range from (i) a melting temperature (Tm) of nucleic acids having a high homology (e.g., a perfectly-matched hybrid) to (ii) a temperature 15°C lower, preferably a temperature 10°C lower, and even preferably a temperature 5°C lower than the melting temperature (Tm).

In one example of the stringent condition, hybridization can be carried out in a buffer solution (including 0.25M Na₂HPO₄, pH 7.2, 7% SDS, 1 mM EDTA, and 1 × Denhardt's solution) for 16 hours to 24 hours at a temperature in a range from 60°C to 68°C, preferably at 65°C, further preferably at 68°C, and then washing can be carried out twice in a buffer solution (including 20 mM Na₂HPO₄, pH 7.2, 1% SDS, and 1 mM EDTA) for 15 minutes at a temperature in a range from 60°C to 68°C, preferably at 65°C, further preferably at 68°C.

In another example, prehybridization is carried out overnight at 42°C in a hybridization solution (including 25% formamide or 50% formamide (for a severer condition), 4 × SSC (sodium chloride/sodium citrate), 50 mM Hepes pH 7.0, 10 × Denhardt's solution, and 20 pg/ml denatured salmon sperm DNA), and then hybridization is carried out by adding a labeled probe thereto and keeping a resulting solution at 42°C overnight. In washing following the hybridization, conditions for a washing solution and a temperature are approximately " 1 × SSC, 0.1% SDS, 37°C", approximately "0.5 × SSC, 0.1% SDS, 42°C" for a severer condition, approximately "0.2 × SSC, 0.1% SDS, 65°C" for a further severer condition. As such, as the conditions for the washing following the hybridization become severer, the specificity of hybridization becomes higher. However, the above-indicated combinations of conditions on SSC, SDS, and temperature are merely examples. A person skilled in the art can provide a stringency similar to the above by appropriately combining the above-described or other elements (e.g., a probe concentration, a probe length, and a time period for a hybridization reaction) that determine the stringency of hybridization. This is disclosed in, for example, Sambrook et al., Molecular Cloning, A Laboratory Manual, 3rd Ed., Cold Spring Harbor Laboratory (2001).

With regard to (5) above, the identity to the amino acid sequence is preferably not less than 92%, more preferably not less than 95%, and even more preferably not less than 99%. The identity to the amino acid sequence may be determined with use of, for example, a program for BLASTX (Altschul et al. J. Mol. Biol., 215: 403-410, 1990). This program is based on the algorithm BLAST by Karlin and Altschul (Proc. Natl. Acad. Sci. USA, 87:2264-2268, 1990, Proc. Natl. Acad. Sci. USA, 90: 5873-5877, 1993).

With regard to (6) above, the phrase "at least one amino acid is deleted, substituted, or added" is intended to mean that a certain number of amino acids which can be substituted, deleted, inserted, or added by a known method for the construction of a variant protein, such as site-directed mutagenesis, are substituted, deleted, inserted, or added. The number of the at least one amino acid is preferably not more than 10, more preferably not more than 5, and even more preferably 1. Note that, in the amino acid sequence, a position where at least one amino acid is deleted, substituted, inserted, or added is not limited.

With respect to (2), (3), (5), and (6) above, whether or not a polypeptide has been expressed on a cell surface can be determined in accordance with a known method (for example, a method described in Example 1 described later). Specifically, for example, a fusion protein of a polypeptide and a green fluorescent protein (GFP) is expressed in a cellulose-synthesizing bacterium or the like. Next, after it has been confirmed that fluorescence is observed, an enzyme capable of decomposing a protein, such as proteinase K, is added to observe a change in fluorescence intensity. In a case where the fluorescence intensity is decreased by the enzyme, it is possible to determine that a polypeptide has been expressed on a cell surface.

The transformant in accordance with another embodiment of the present invention is obtained by introducing an oprI gene into a bacterium belonging to the family *Methylobacteriaceae.* The present transformant contains an oprI gene and thus enables a desired protein to be expressed on a bacterial cell surface. Any technique for causing a bacterium belonging to the family *Methylobacteriaceae*, which is a cellulose-synthesizing bacterium, to express a protein on a bacterial cell surface did not conventionally exist. Further, by expressing, for example, cellulase (described later) as the protein, it is possible to produce glucose or cellobiose from cellulose without adding cellulase from the outside. In addition, it is possible to produce glucose or cellobiose with high productivity (specific productivity) per bacterial cell and at higher yield. Note that the oprI gene is as described above.

The transformant in accordance with another embodiment of the present invention is preferably obtained by further introducing a cellulase gene. In a case where cellulase is added to a cellulose-synthesizing bacterium to produce glucose, cellulase needs to be continuously added from the outside. However, when a cellulase gene is introduced into the present transformant, cellulase is expressed on a bacterial cell surface. Thus, it is possible to maintain cellulase activity without adding cellulase.

The cellulase is not particularly limited, provided that it is capable of decomposing cellulose. Examples of the cellulase includes endoglucanase, exoglucanase, β-glucosidase, and the like. In the present transformant, only one type of cellulase among the above-described cellulases may be expressed, or a plurality of types of cellulases among the above-described cellulases may be expressed.

The present transformant preferably contains, as the cellulase gene, at least one selected from an endoglucanase gene and an exoglucanase gene. The present transformant more preferably contains both an endoglucanase gene and an exoglucanase gene.

In an embodiment of the present invention, the exoglucanase gene is preferably at least one DNA selected from the group consisting of the following (1) to (6):
(1) DNA consisting of a base sequence of SEQ ID NO: 5;
(2) DNA encoding a protein that has not less than 90% homology to the base sequence of SEQ ID NO: 5 and that has exoglucanase activity;
(3) DNA (i) hybridizing, under a stringent condition, with a gene consisting of the base sequence of SEQ ID NO: 5 and (ii) encoding a protein that has exoglucanase activity;
(4) DNA encoding a polypeptide consisting of an amino acid sequence of SEQ ID NO: 11;
(5) DNA encoding a protein that has not less than 90% identity to the amino acid sequence of SEQ ID NO: 11 and that has exoglucanase activity; and
(6) DNA encoding a protein (i) consisting of an amino acid sequence in which at least one amino acid is deleted, substituted, inserted, or added in the amino acid sequence of SEQ ID NO: 11 and (ii) having exoglucanase activity.

The SEQ ID NO: 5 corresponds to the full-length base sequence of the cex gene used in Examples. Further, the SEQ ID NO: 11 corresponds to the full-length amino acid sequence encoded by the cex gene.

In an embodiment of the present invention, the endoglucanase gene is preferably at least one DNA selected from the group consisting of the following (1) to (6):
(1) DNA consisting of a base sequence of SEQ ID NO: 6;
(2) DNA encoding a protein that has not less than 90% homology to the base sequence of SEQ ID NO: 6 and that has endoglucanase activity;
(3) DNA (i) hybridizing, under a stringent condition, with a gene consisting of the base sequence of SEQ ID NO: 6 and (ii) encoding a protein that has endoglucanase activity;
(4) DNA encoding a polypeptide consisting of an amino acid sequence of SEQ ID NO: 12;
(5) DNA encoding a protein that has not less than 90% identity to the amino acid sequence of SEQ ID NO: 12 and that has endoglucanase activity; and
(6) DNA encoding a protein (i) consisting of an amino acid sequence in which at least one amino acid is deleted, substituted, inserted, or added in the amino acid sequence of SEQ ID NO: 12 and (ii) having endoglucanase activity.

The SEQ ID NO: 6 corresponds to the full-length base sequence of the cenA gene used in Examples. Further, the SEQ ID NO: 12 corresponds to the full-length amino acid sequence encoded by the cenA gene.

The above-described configuration makes it possible to efficiently decompose cellulose synthesized by the present transformant so that cellobiose is produced.

The present transformant preferably further contains a β-glucosidase gene as the cellulase gene. Further, the β-glucosidase gene is preferably at least one DNA selected from the group consisting of the following (1) to (6):
(1) DNA consisting of a base sequence of SEQ ID NO: 4;
(2) DNA encoding a protein that has not less than 90% homology to the base sequence of SEQ ID NO: 4 and that has β-glucosidase activity;
(3) DNA (i) hybridizing, under a stringent condition, with a gene consisting of the base sequence of SEQ ID NO: 4 and (ii) encoding a protein that has β-glucosidase activity;
(4) DNA encoding a polypeptide consisting of an amino acid sequence of SEQ ID NO: 10;
(5) DNA encoding a protein that has not less than 90% identity to the amino acid sequence of SEQ ID NO: 10 and that has β-glucosidase activity; and
(6) DNA encoding a protein (i) consisting of an amino acid sequence in which at least one amino acid is deleted, substituted, inserted, or added in the amino acid sequence of SEQ ID NO: 10 and (ii) having β-glucosidase activity.

The SEQ ID NO: 4 corresponds to the full-length base sequence of the bglC gene used in Examples. Further, the SEQ ID NO: 10 corresponds to the full-length amino acid sequence encoded by the bglC gene.

The above-described configuration makes it possible to efficiently decompose cellobiose produced by the endoglucanase and/or exoglucanase so that glucose is produced. The details of (1) to (6) relating to the endoglucanase gene, the exoglucanase gene, and the β-glucosidase gene are the same as the matters described for (1) to (6) relating to the oprI gene described above.

### [2. Method for producing glucose or cellobiose]

A method for producing glucose or cellobiose in accordance with an embodiment of the present invention includes a culture step of culturing the present transformant with use of a carbon source that is utilizable by the present transformant. According to the above-described production method, it is not necessary to add cellulase or the like, and it is thus possible to produce glucose or cellobiose more efficiently than before. In addition, the present transformant can produce glucose or cellobiose with higher productivity (specific productivity) per cell (bacterial cell) and at higher yield than before.

Examples of the carbon source that is utilizable by the present transformant include methanol, ethanol, L-malic acid, L-lactic acid, succinic acid, methylamine, fumaric acid, formic acid, acetic acid, betaine, and the like, and salts thereof. Among these, the carbon source that is utilizable by the present transformant is preferably L-malic acid, L-lactic acid, succinic acid, methylamine, fumaric acid, and salts thereof, and methanol and ethanol, all of which are carbon sources suitable for culture, and is more preferably methanol from the viewpoint of commercial production of a CO₂-derived product, as described above. The type of salt is not particularly limited, and may be, for example, a sodium salt or a hydrochloride.

In the culture step, a condition for culturing the present transformant can be set as appropriate according to the type of the present transformant. Such a culture condition is known to a person skilled in the art. A culture medium may include, in addition to a carbon source, a nitrogen source, an inorganic salt, or the like as needed, and may be any of a natural medium and a synthetic medium. The culture medium may be, for example, an MP medium described in PLoS ONE 8(4):e62957 (2013).

The culture step is preferably carried out under an aerobic condition such as shaking culture or aeration stirring culture. A culture temperature may be, for example, 20°C to 40°C, the pH of the culture medium may be 5 to 9, and a culture time may be, for example, 0 to 13 days.

Whether sugar to be produced by the above-described production method is glucose or cellobiose can be determined by a combination of cellulase genes introduced into the above-described present transformant. In a case where glucose is desired to be produced, for example, in addition to an endoglucanase gene or an exoglucanase gene, a β-glucosidase gene are introduced. In a case where cellobiose is desired to be produced, the introduction of the β-glucosidase gene is omitted.

### [3. Method for producing composition containing glucose or cellobiose]

A method for producing a composition in accordance with an embodiment of the present invention includes a step of producing glucose or cellobiose by the above-described production method. That is, the composition contains glucose or cellobiose obtained by the above-described production method. Examples of such a composition include: a cultured product containing glucose or cellobiose and the present transformant; a cultured product obtained by co-culturing, with the present transformant, a bacterium utilizing, as a nutrient source, glucose or cellobiose or a transformant of such a bacterium other than the present transformant; and the like. The composition containing glucose or cellobiose may further contain any of utilizable carbon sources including methanol, ethanol, L-malic acid, L-lactic acid, succinic acid, methylamine, fumaric acid, formic acid, acetic acid, betaine, and the like, and salts thereof.

Note that it is impossible or impractical to directly identify the above-described composition by its structure or by its property. Glucose and cellobiose produced by the above-described production method cannot be directly identified by its structure or by its property, in light of the fact that distinguishing, on the basis of a structure or a property, from glucose and cellobiose produced by other production method is impossible.

The concentration of glucose or cellobiose in the composition containing glucose or cellobiose is preferably 0.05 g/L to 0.5 g/L, and more preferably 0.1 g/L to 0.4 g/L.

### [4. Method for producing article]

A method for producing an article in accordance with an embodiment of the present invention includes a step of producing glucose or cellobiose by the above-described production method. The article is not particularly limited, provided that it is an article containing glucose or cellobiose or an article produced with use of glucose or cellobiose. Examples of the article include food, feed, a nutrient source for a microorganism, a raw material for a chemical product, and the like.

Note that the method for producing an article may further include a step of producing an article (for example, food, feed, a nutrient source for a microorganism, a raw material for a chemical product, and the like) with use of glucose or cellobiose produced by the above-described production method. In addition, in the method for producing an article, a composition containing glucose or cellobiose produced by the above-described production method may be used.

Examples of the food, the feed, or the nutrient source for a microorganism include: a protein source and a sugar source obtained by dry-solidifying a cultured product containing glucose or cellobiose and a transformant as it is; a purified sugar source obtained by removing impurities such as a transformant from the cultured product; and the like. The food, the feed, or the nutrient source for a microorganism may further contain an arbitrary product produced with use of glucose or cellobiose as a nutrient source by a bacterium or a transformant of the bacterium other than the present transformant. Note that the arbitrary product can be changed as appropriate according to food, feed, or a nutrient source for a microorganism.

Examples of the raw material for a chemical product include: a plastic raw material obtained by chemically converting glucose or cellobiose which has been purified from a cultured product; and the like. It is preferable that the raw material for a chemical product do not contain impurities (such as the present transformant) other than glucose or cellobiose.

The present invention is not limited to the embodiments, but can be altered by a skilled person in the art within the scope of the claims. The present invention also encompasses, in its technical scope, any embodiment derived by combining technical means disclosed in differing embodiments.

### <Others>

< 1>
   A transformant obtained by introducing a cellulase gene and a cell surface protein gene into a cellulose-synthesizing microorganism.
<2>
   The transformant described in <1>, wherein the cellulose-synthesizing microorganism is a cellulose-synthesizing bacterium.
<3>
   The transformant described in <2>, wherein the cellulose-synthesizing bacterium is a bacterium belonging to the family *Methylobacteriaceae.*
<4>
   The transformant described in <3>, wherein the bacterium belonging to the family *Methylobacteriaceae* is a bacterium belonging to the genus *Methylorubrum.*
<5>
   The transformant described in <4>, wherein the bacterium belonging to the genus *Methylorubrum* is *Methylorubrum extorquens.*
<6>
   The transformant described in any of <1> to <5>, wherein the cell surface protein gene is an oprI gene.
<7>
   A transformant obtained by introducing an oprI gene into a bacterium belonging to the family *Methylobacteriaceae.*
<8>
   The transformant described in <7>, wherein the bacterium belonging to the family *Methylobacteriaceae* is a bacterium belonging to the genus *Methylorubrum.*
<9>
   The transformant described in <8>, wherein the bacterium belonging to the genus *Methylorubrum* is *Methylorubrum extorquens.*
<10> The transformant described in any of <6> to <9>, wherein the oprI gene is any DNA selected from the group consisting of the following (1) to (6):
   (1) DNA consisting of a base sequence of SEQ ID NO: 1;
   (2) DNA encoding a polypeptide that has not less than 90% homology to the base sequence of SEQ ID NO: 1 and that is a cell surface protein;
   (3) DNA (i) hybridizing, under a stringent condition, with a gene consisting of a base sequence complementary to the base sequence of SEQ ID NO: 1 and (ii) encoding a polypeptide that is a cell surface protein;
   (4) DNA encoding a polypeptide consisting of an amino acid sequence of SEQ ID NO: 2;
   (5) DNA encoding a polypeptide that has not less than 90% identity to the amino acid sequence of SEQ ID NO: 2 and that is a cell surface protein; and
   (6) DNA encoding a polypeptide (i) consisting of an amino acid sequence in which at least one amino acid is deleted, substituted, inserted, or added in the amino acid sequence of SEQ ID NO: 2 and (ii) being a cell surface protein.
<11> The transformant described in any of <7> to <10>, obtained by further introducing a cellulase gene.
<12>
   The transformant described in any of <1> to <6> and <11>, wherein at least one selected from an endoglucanase gene and an exoglucanase gene is contained as the cellulase gene.
<13>
   The transformant described in <12>, wherein the exoglucanase gene is at least one DNA selected from the group consisting of the following (1) to (6):
   (1) DNA consisting of a base sequence of SEQ ID NO: 5;
   (2) DNA encoding a protein that has not less than 90% homology to the base sequence of SEQ ID NO: 5 and that has exoglucanase activity;
   (3) DNA (i) hybridizing, under a stringent condition, with a gene consisting of the base sequence of SEQ ID NO: 5 and (ii) encoding a protein that has exoglucanase activity;
   (4) DNA encoding a polypeptide consisting of an amino acid sequence of SEQ ID NO: 11;
   (5) DNA encoding a protein that has not less than 90% identity to the amino acid sequence of SEQ ID NO: 11 and that has exoglucanase activity; and
   (6) DNA encoding a protein (i) consisting of an amino acid sequence in which at least one amino acid is deleted, substituted, inserted, or added in the amino acid sequence of SEQ ID NO: 11 and (ii) having exoglucanase activity.
< 14>
   The transformant described in <12> or <13>, wherein the endoglucanase gene is at least one DNA selected from the group consisting of the following (1) to (6):
   (1) DNA consisting of a base sequence of SEQ ID NO: 6;
   (2) DNA encoding a protein that has not less than 90% homology to the base sequence of SEQ ID NO: 6 and that has endoglucanase activity;
   (3) DNA (i) hybridizing, under a stringent condition, with a gene consisting of the base sequence of SEQ ID NO: 6 and (ii) encoding a protein that has endoglucanase activity;
   (4) DNA encoding a polypeptide consisting of an amino acid sequence of SEQ ID NO: 12;
   (5) DNA encoding a protein that has not less than 90% identity to the amino acid sequence of SEQ ID NO: 12 and that has endoglucanase activity; and
   (6) DNA encoding a protein (i) consisting of an amino acid sequence in which at least one amino acid is deleted, substituted, inserted, or added in the amino acid sequence of SEQ ID NO: 12 and (ii) having endoglucanase activity.
<15>
   The transformant described in any of <12> to <14>, wherein a β-glucosidase gene is further contained as the cellulase gene.
<16>
   The transformant described in <15>, wherein the β-glucosidase gene is at least one DNA selected from the group consisting of the following (1) to (6):
   (1) DNA consisting of a base sequence of SEQ ID NO: 4;
   (2) DNA encoding a protein that has not less than 90% homology to the base sequence of SEQ ID NO: 4 and that has β-glucosidase activity;
   (3) DNA (i) hybridizing, under a stringent condition, with a gene consisting of the base sequence of SEQ ID NO: 4 and (ii) encoding a protein that has β-glucosidase activity;
   (4) DNA encoding a polypeptide consisting of an amino acid sequence of SEQ ID NO: 10;
   (5) DNA encoding a protein that has not less than 90% identity to the amino acid sequence of SEQ ID NO: 10 and that has β-glucosidase activity; and
   (6) DNA encoding a protein (i) consisting of an amino acid sequence in which at least one amino acid is deleted, substituted, inserted, or added in the amino acid sequence of SEQ ID NO: 10 and (ii) having β-glucosidase activity.
< 17>
   A method for producing glucose or cellobiose, including a culture step of culturing a transformant described in any of <1> to <16> with use of a carbon source that is utilizable by the transformant.
<18>
   A method for producing an article, including a step of producing glucose or cellobiose by the method described in < 17>.
<19>
   A composition including glucose or cellobiose obtained by the method described in < 17>.

### Examples

The following will describe Examples of the present invention. However, the present invention is not limited by these Examples.

### [Example 1: Bacterial cell surface expression of green fluorescent protein (GFP)]

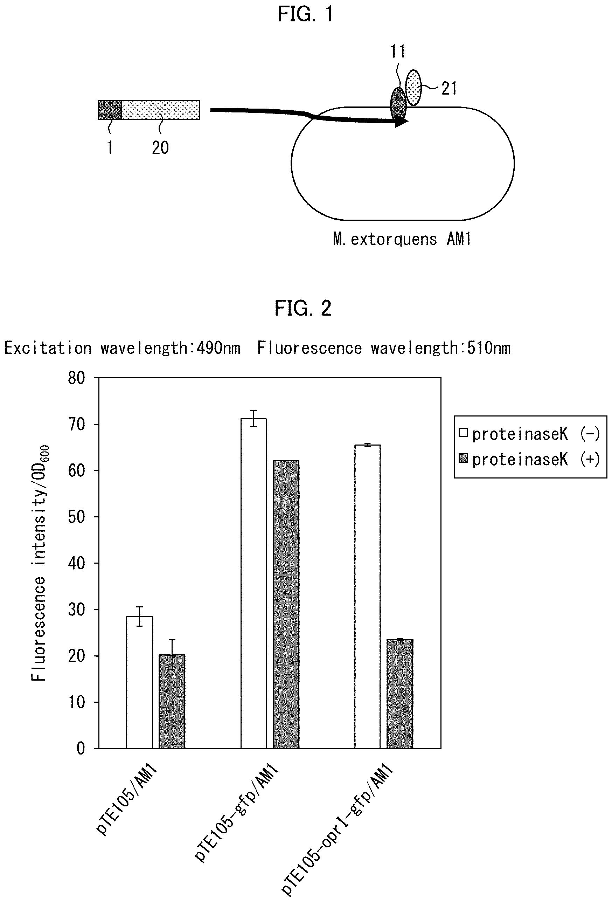

Fig. 1 is a schematic diagram of a transformant prepared in Example 1. As illustrated in Fig. 1, prepared in Example 1 was a transformant obtained by introducing, into a cellulose-synthesizing bacterium, a gene obtained by binding an oprI gene 1 and a GFP gene 20 to each other. In the transformant, a GFP 21 is expressed on a bacterial cell surface by an oprI protein 11. The following will specifically describe a method for preparing the transformant and test results obtained by using the transformant.

### <1-1. Construction of plasmid>

### (pTE 105-gfp)

With use of pTE105 (available from Addgene) as a template and with use of pTE105-KpnI-F1 (SEQ ID NO: 13) and pTE105-SpeI-Rl (SEQ ID NO: 14) as primers, pTE105 was amplified by PCR. The PCR was carried out under the following conditions: 95°C for 10 seconds; 35 cycles of (95°C for 10 seconds, 70°C for 30 seconds, and 72°C for 4 minutes); and 72°C for 2 minutes. With use of pGreenTIR (Gene 191(2): 149-153 (1997)) as a template and with use of gfp-SpeI-RBS-F1 (SEQ ID NO: 15) and gfp-KpnI-R1 (SEQ ID NO: 16) as primers, gfp (SEQ ID NO: 3) was amplified by PCR. The PCR was carried out under the following conditions: 95°C for 10 seconds; 35 cycles of (95°C for 10 seconds, 70°C for 30 seconds, and 72°C for 4 minutes); and 72°C for 2 minutes. These PCR amplified fragments were purified by gel extraction, and respective purified products of the amplified fragments were mixed in equimolar proportions. After that, a resulting mixture was mixed with NEBuilder HiFi DNA Assembly Master Mix (NEB) in equal amounts. A resulting mixture was incubated at 55°C for 15 minutes to obtain a reaction product. With use of 5 pL of the reaction product thus obtained, *Escherichia coli* DH5α was transformed by a heat shock method. After the transformation has been finished, the transformed *Escherichia coli* DH5α was cultured in an LB agar medium (20 pg/mL tetracycline added) to obtain a transformant having a sequence of pTE105-gfp (SEQ ID NO: 3). The plasmid was extracted from the transformant with use of MagExtractor -Plasmid- (Toyobo), and it was confirmed by electrophoresis that the extracted plasmid was a target plasmid pTE105-gfp.

### (pTE 105-oprI-gfp)

With use of pTE105 (available from Addgene) as a template and with use of pTE105-KpnI-F1 (SEQ ID NO: 13) and pTE105-SpeI-Rl (SEQ ID NO: 14) as primers, pTE105 was amplified by PCR. The PCR was carried out under the following conditions: 95°C for 10 seconds; 35 cycles of (95°C for 10 seconds, 70°C for 30 seconds, and 72°C for 4 minutes); and 72°C for 2 minutes. With use of pVUB3 (Gene 221: 25-34 (1998)) as a template and with use of oprI-SpeI-RBS-F1 (SEQ ID NO: 17) and oprI-R1 (SEQ ID NO: 18) as primers, oprI (SEQ ID NO: 1) was amplified by PCR. The PCR was carried out under the following conditions: 95°C for 10 seconds; 35 cycles of (95°C for 10 seconds, 60°C for 30 seconds, and 72°C for 4 minutes); and 72°C for 2 minutes. With use of pGreenTIR (Gene 191: 149-153 (1997)) as a template and with use of gfp-F2 (SEQ ID NO: 19) and gfp-KpnI-R1 (SEQ ID NO: 16) as primers, gfp (SEQ ID NO: 3) was amplified by PCR. The PCR was carried out under the following condition: 95°C for 10 seconds, 35 cycles of (95°C for 10 seconds, 70°C for 30 seconds, and 72°C for 4 minutes), and 72°C for 2 minutes. These PCR amplified fragments were purified by gel extraction, and respective purified products of the amplified fragments were mixed in equimolar proportions. After that, a resulting mixture was mixed with NEBuilder HiFi DNA Assembly Master Mix (NEB) in equal amounts. A resulting mixture was incubated at 55°C for 15 minutes to obtain a reaction product. With use of 5 pL of the reaction product, E. *coli* DH5α was transformed by a heat shock method. After the transformation had been finished, the transformed E. *coli* DH5α was cultured in an LB agar medium (20 pg/mL tetracycline added) to obtain a transformant having a sequence of pTE 105-oprI-gfp (SEQ ID NO: 7). The plasmid was extracted from the transformant with use of MagExtractor -Plasmid- (Toyobo), and it was confirmed by electrophoresis that the extracted plasmid was a target plasmid pTE 105-oprI-gfp.

### < 1-2. Preparation of genetically modified organism>

*Methylorubrum extorquens* AM1 (JCM 2805, available from Riken BioResource Research Center) was transformed with use of each of the following plasmids: the plasmid pTE105; and the plasmids pTE105-gfp and pTE105-oprI-gfp, which had been constructed in < 1-1. Construction of plasmid>. The transformation was carried out by an electroporation method (FEMS microbiol. Lett. 166(1): 1-7 (1998)). After the transformation had been finished, culturing was carried out in a 1 vol% methanol-containing MP agar medium (20 pg/mL tetracycline added) to obtain transformants *Methylorubrum extorquens* AM1 (pTE105), M. *extorquens* AM1 (pTE105-gfp), and M. *extorquens* AM1 (pTE105-oprI-gfp). The plasmids were extracted from the corresponding transformants with use of MagExtractor -Plasmid- (Toyobo), and it was confirmed by electrophoresis that the extracted plasmids were target plasmids pTE105-gfp and pTE 105-oprI-gfp. Note that the composition of the 1 vol% methanol-containing MP medium is as follows. Content per liter: 10 mL of methanol, 9.1 g of PIPES, 0.3 g of K₂HPO₄, 0.3 g of NaH₂PO₄2H₂O, 0.1 g of MgCl₂·6H₂O, 2.6 g of (NH₄)₂SO₄, 2.2 mg of CaCl₂, 13.4 mg of Na₃C₆H₅O₇·2H₂O, 0.3 mg of ZnSO₄·7H₂O, 0.2 mg of MnCl₂·4H₂O, 5 mg of FeSO₄·7H₂O, 2.5 mg of (NH₄)₆M_{O7}O₂₄·4H₂O, 0.3 mg of CuSO₄·5H₂O, 0.5 mg of CoCl₂·6H₂O, 0.1 mg of Na₂WO₄·2H₂O) (PLoS ONE 8(4): e62957 (2013)).

### < 1 -3. Expression of GFP>

The transformants *Methylorubrum extorquens* AM1 (pTE105), *M. extorquens* AM1 (pTE 105-gfp), and M. *extorquens* AM1 (pTE105-oprI-gfp), which had been obtained in <1-2. Preparation of genetically modified organism>, were each inoculated into 50 mL of a 1 vol% methanol-containing MP medium (20 pg/mL tetracycline, 0.3 U/mL β-glucosidase (Toyobo), and 2 U/mL cellulase (Nacalai Tesque) added), and pre-culturing was carried out at 30°C at a shake speed of 150 rpm for 5 days. After the bacterial cells had been collected from a culture solution obtained by the pre-culturing, the bacterial cells were washed three times with use of physiological saline. The bacterial cells were inoculated into 50 mL of the MP medium such that the culture solution turbidity (OD₆₀₀) at a wavelength of 600 nm became 0.1, and main culturing was carried out at 30°C at a shake speed of 150 rpm for 5 days. After the bacterial cells had been collected from 1 mL of a main culture solution, the bacterial cells were washed three times with use of physiological saline. After the washing, the bacterial cells were resuspended in 1 mL of 100 mM sodium phosphate buffer solution (pH 7.0) or 1 mL of 100 mM sodium phosphate buffer solution to which 1 mg/mL proteinase K had been added. After incubation was carried out at 37°C for 18 hours, the bacterial cells were collected, and the bacterial cells were washed three times with use of physiological saline. Resulting collected bacterial cells were resuspended in 1 mL of 100 mM sodium phosphate buffer solution (pH 7.0). Fluorescence intensity of each bacterial cell suspension was examined at an excitation wavelength of 490 nm and a fluorescence wavelength of 510 nm with use of a fluorophotometer FP6500 (JASCO Corporation). The results are shown in Fig. 2.

In Fig. 2, *M. extorquens* AM 1 (pTE105-gfp) and M. *extorquens* AM 1 (pTE 105-oprI-gfp) into which GFP was introduced showed high fluorescence intensity. However, in a case where proteinase K was added, only M. *extorquens* AM 1 (pTE 105-oprI-gfp) showed decreased fluorescence intensity. This is considered to have occurred because GFP was expressed on the bacterial cell surface by oprI, and GFP was decomposed by proteinase K. Therefore, it was shown that adding oprI to a protein-expressing gene enables a protein to be expressed on a bacterial cell surface.

### [Example 2: Glucose synthesis with use of methanol as raw material]

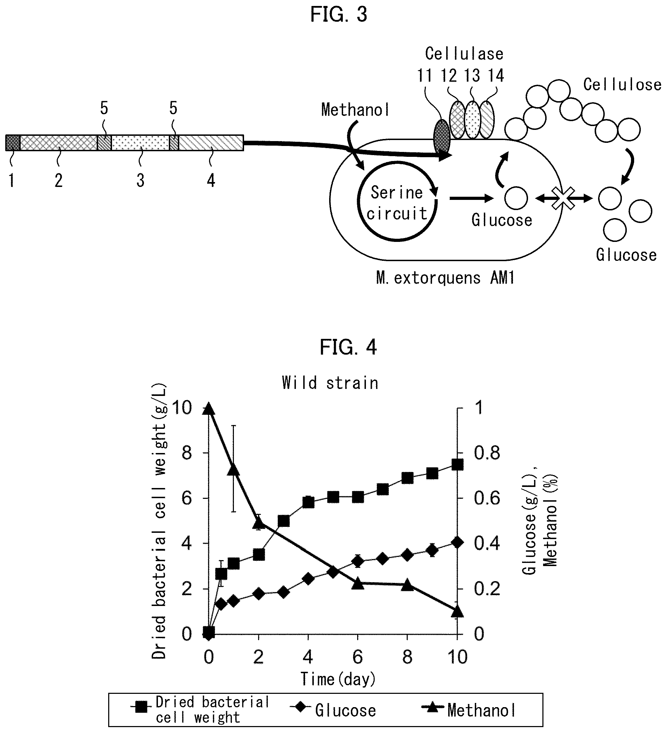

Fig. 3 is a schematic diagram of a transformant prepared in Example 2. As illustrated in Fig. 3, prepared in Example 2 was a transformant obtained by introducing, into a cellulose-synthesizing bacterium, a gene obtained by binding an oprI gene 1 and cellulase-expressing genes, which are a β-glucosidase gene 2, an exoglucanase gene 3, and an endoglucanase gene 4, to each other via a linker 5. In the transformant, β-glucosidase 12, exoglucanase 13, and endoglucanase 14 are expressed on a bacterial cell surface by an oprI protein 11. Therefore, cellulose produced from glucose inside a bacterial cell by the transformant is decomposed by cellulase that has been expressed on the bacterial cell surface, and glucose is produced outside the bacterial cell. The following will specifically describe a method for preparing the transformant and test results obtained by using the transformant.

### <2-1. Construction of plasmid>

### (pTE 105-oprI-bglC-cex-cenA)

With use of pTE105-oprI-gfp, which had been prepared in Example 1, as a template and with use of pTE 1 05-KpnI-F 1 (SEQ ID NO: 13) and oprI-R2 (SEQ ID NO: 26) as primers, pTE105-oprI was amplified by PCR. The PCR was carried out under the following condition: 95°C for 10 seconds, 35 cycles of (95°C for 10 seconds, 70°C for 30 seconds, and 72°C for 4 minutes), and 72°C for 2 minutes. With use of pGV3-bglC (J. Biosci. Bioeng. 127(4): 441-446 (2019)) as a template and with use of bglC-F1 (SEQ ID NO: 20) and bglC-R1 (SEQ ID NO: 21) as primers, bglC (SEQ ID NO: 4) was amplified by PCR. The PCR was carried out under the following conditions: 95°C for 10 seconds; 35 cycles of (95°C for 10 seconds, 60°C for 30 seconds, and 72°C for 4 minutes); and 72°C for 2 minutes. With use of genomic DNA of *Cellulomonas fimi* NBRC15513 (available from the National Institute of Technology and Evaluation) as a template and with use of cex-F1 (SEQ ID NO: 22) and cex-R1 (SEQ ID NO: 23) as primers, cex (SEQ ID NO: 5) was amplified by PCR. The PCR was carried out under the following conditions: 95°C for 10 seconds; 35 cycles of (95°C for 10 seconds, 60°C for 30 seconds, and 72°C for 4 minutes); and 72°C for 2 minutes. With use of genomic DNA of *C*. *fimi* NBRC15513 as a template and with use of cenA-F1 (SEQ ID NO: 24) and cenA-R1 (SEQ ID NO: 25) as primers, cenA (SEQ ID NO: 6) was amplified by PCR. The PCR was carried out under the following conditions: 95°C for 10 seconds; 35 cycles of (95°C for 10 seconds, 60°C for 30 seconds, and 72°C for 4 minutes); and 72°C for 2 minutes. These PCR amplified fragments were purified by gel extraction, and respective purified products of the amplified fragments were mixed in equimolar proportions. After that, a resulting mixture was mixed with NEBuilder HiFi DNA Assembly Master Mix (NEB) in equal amounts. A resulting mixture was incubated at 55°C for 15 minutes to obtain a reaction product. With use of 5 pL of the reaction product, *E*. *coli* DH5α was transformed by a heat shock method. After the transformation had been finished, the transformed *E*. *coli* DH5α was cultured in an LB agar medium (20 µg/mL tetracycline added) to obtain a transformant having a sequence of oprI-bglC-cex-cenA (SEQ ID NO: 8). The plasmid was extracted from the transformant with use of MagExtractor -Plasmid- (Toyobo), and it was confirmed by electrophoresis that the extracted plasmid was a target plasmid pTE 105-oprI-bglC-cex-cenA (hereinafter also referred to as pTE105-oprI-bcc).

### <2-2. Preparation of genetically modified organism>

*M. extorquens* AM1 (JCM 2805, available from Riken BioResource Research Center) was transformed with use of each of the following plasmids: the plasmid pTE105; and the plasmid pTE 105-oprI-bcc, which had been constructed in <2-1. Construction of plasmid>. The transformation was carried out by an electroporation method (FEMS microbiol. Lett. 166(1): 1-7 (1998)). After the transformation had been finished, culturing was carried out in a 1 vol% methanol-containing MP agar medium (20 pg/mL tetracycline added) to obtain transformants *M*. *extorquens* AM1 (pTE105) and *M.extorquens* AM1 (pTE105-oprI-bcc). Note that the composition of the 1 vol% methanol-containing MP medium is identical to the 1 vol% methanol-containing MP medium used in < 1-2. Preparation of genetically modified organism>.

### <2-3. Glucose production from methanol>

*M. extorquens* AM1 (pTE 105-oprI-bcc) was inoculated into a medium which had been obtained by adding 20 µg/mL tetracycline to 50 mL of a 1 vol% methanol-containing MP medium. In addition, *M. extorquens* AM1 (wild strain) was inoculated into a medium which had been obtained by adding 0.3 U/mL β-glucosidase (Toyobo) and 2 U/mL cellulase (Nacalai Tesque) to 50 mL of a 1 vol% methanol-containing MP medium. After *M*. *extorquens* AM 1 (pTE 105-oprI-bcc) and M. *extorquens* AM 1 (wild strain) had been inoculated into the corresponding media, pre-culturing was carried out at 30°C at a shake speed of 150 rpm for 5 days. After the bacterial cells had been collected from a culture solution obtained by the pre-culturing, the bacterial cells were washed three times with use of physiological saline. The bacterial cells were inoculated into a medium having the same composition as 50 mL of the medium used in the pre-culturing such that the culture solution turbidity (OD₆₀₀) at 600 nm was 0.1, and main culturing was carried out at 30°C at a shake speed of 150 rpm for 10 days or 13 days. However, for the wild strain, 0.3 U/mL β-glucosidase (Toyobo) and 2 U/mL cellulase (Nacalai Tesque) were added to the culture solution every 2 days.

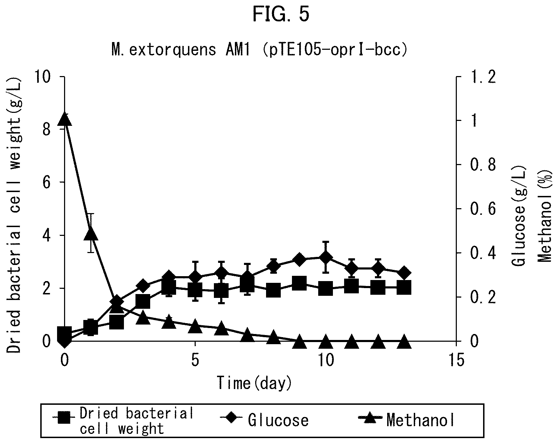

Glucose was quantified with use of glucose CII-Test Wako (Wako Pure Chemical Industries, Ltd.). After 2 pL of a supernatant of the culture solution and 300 pL of a coloring test solution had been mixed, a resulting mixture was reacted at room temperature for 15 minutes, and the absorbance at a wavelength of 505 nm was measured. Methanol was quantified with use of a gas chromatograph GC-2025 system (Shimadzu Corporation). A column was MCI GEL CRS 10W (Mitsubishi Chemical), a detector was an FID detector, a mobile phase was nitrogen gas, a flow velocity was 0.5 mL/min, a column temperature was 50°C, a detector temperature was 50°C, and an injection port temperature was 250°C. The results of the measurements are shown in Figs. 4 and 5.

Fig. 4 shows the result obtained by using the wild strain, and Fig. 5 shows the result obtained by using M. *extorquens* AM 1 (pTE 105-oprI-bcc). From Fig. 5, it is clear that *M. extorquens* AM 1 (pTE 105-oprI-bcc) is capable of producing glucose without addition of β-glucosidase and cellulase. Further, in Figs. 4 and 5, in the case of the wild strain, a dried bacterial cell weight increases over time, but, in the case of M. *extorquens* AM 1 (pTE 105-oprI-bcc), a bacterial cell weight does not increase. Thus, it is clear that, as compared to the wild strain, *M. extorquens* AM 1 (pTE 105-oprI-bcc) has high glucose productivity (specific productivity) per bacterial cell and is capable of producing glucose at high yield. In addition, as compared to the wild strain in Fig. 4, *M. extorquens* AM1 (pTE 105-oprI-bcc) in Fig. 5 consumes methanol in a shorter time and produces glucose in a shorter time. Thus, it is also clear that *M*. *extorquens* AM 1 (pTE 105-oprI-bcc) has an excellent glucose production speed.

### <2-4. Cellulase activity>

The wild strain was inoculated into 50 mL of a 1 vol% methanol-containing MP medium, *M. extorquens* AM 1 (pTE 105) and *M*. *extorquens* AM 1 (pTE 105-oprI-bcc) described above were each inoculated into 50 mL of a 1 vol% methanol-containing MP medium (20 pg/mL tetracycline added), and pre-culturing was carried out at 30°C at a shake speed of 150 rpm for 5 days. After the bacterial cells had been collected from a culture solution obtained by the pre-culturing, the bacterial cells were washed three times with use of physiological saline. The bacterial cells were inoculated into a medium having the same composition as 50 mL of the medium used in the pre-culturing such that the concentration became 1 vol%, and main culturing was carried out at 30°C at a shake speed of 150 rpm for 5 days. After the bacterial cells had been collected from 1 mL of a main culture solution, the bacterial cells were washed three times with use of physiological saline. After the washing, the bacterial cells were resuspended in 1 mL of 100 mM sodium phosphate buffer solution (pH 7.0) or 1 mL of 100 mM sodium phosphate buffer solution to which 1 mg/mL proteinase K had been added. The same bacterial cell suspension was used for the activity measurement of three types of cellulases (β-glucosidase, endoglucanase, and exoglucanase).

Further, a change over time of the cellulase activity in the culture solution was also measured. *M. extorquens* AM 1 (wild type) was inoculated into a medium which had been obtained by adding 0.3 U/mL β-glucosidase (Toyobo) and 2 U/mL cellulase (Nacalai Tesque) to 50 mL of a 1 vol% methanol-containing MP medium. In addition, *M*. *extorquens* AM 1 (pTE 105-oprI-bcc) was inoculated into a medium which had been obtained by adding 20 pg/mL tetracycline to 50 mL of a 1 vol% methanol-containing MP medium. After M. *extorquens* AM 1 (wild type) and *M*. *extorquens* AM1 (pTE 105-oprI-bcc) had been inoculated into the corresponding media, pre-culturing was carried out at 30°C at a shake speed of 150 rpm for 5 days. After the bacterial cells had been collected from a pre-culture solution, the bacterial cells were washed three times with use of physiological saline and then suspended in 50 mL of physiological saline. The bacterial cell suspension was inoculated into a medium having the same composition as 50 mL of the medium used in the pre-culturing so as to be become 1 vol%, and main culturing was carried out at 30°C at a shake speed of 150 rpm for 6 days. After the culturing had been finished, a change over time of each enzyme activity in the main culture solution was measured. A method for measuring each enzyme activity is as below.

### (β-glucosidase activity measurement)

500 pL of 10 mM p-nitrophenyl-β-D-glucopyranoside dissolved in a 50 mM sodium phosphate buffer solution (pH 7.0) was added to 10 pL of bacterial cell suspension or 10 pL of culture solution, and incubation was carried out at 30°C. After the incubation, 100 pL of the bacterial cell suspension or 100 pL of the culture solution was separated, 1 mL of a 2 M sodium carbonate aqueous solution was added rapidly, and the reaction was stopped. After the reaction had been stopped, the bacterial cell suspension or the culture solution was centrifuged to collect a supernatant. For the collected supernatant, the amount of p-nitrophenol produced was determined by measuring the absorbance at a wavelength of 400 nm. The enzyme activity was calculated with use of the molar extinction coefficient of p-nitrophenol of 18.5 mM⁻¹ at 400 nm.

### (Endoglucanase activity measurement)

100 pL of 1% carboxymethyl cellulose dissolved in a 50 mM sodium phosphate buffer solution (pH 7.0) was added to 100 pL of bacterial cell suspension or 100 pL of culture solution, and incubation was carried out at 30°C. After the incubation, 100 pL of the bacterial cell suspension or 100 pL of the culture solution was separated, and 300 pL of a 1% 3,5-dinitrosalicylic acid solution (10 g/L, 3,5-dinitrosalicylic acid solution, 10 g/L sodium hydroxide, 2 g/L phenol, and 0.5 g/L sodium hydrogen sulfate) was added. After the addition, the bacterial cell suspension or the culture solution was heated at 100°C for 15 minutes to stop the reaction. After the reaction had been stopped, the bacterial cell suspension or the culture solution to which 300 pL of a 40% potassium sodium tartrate aqueous solution had been added was centrifuged to collect a supernatant. For the collected supernatant, the amount of glucose produced was determined by measuring the absorbance at a wavelength of 540 nm. The enzyme activity was calculated from the amount of glucose produced.

### (Exoglucanase activity measurement)

100 pL of 1% microcrystalline cellulose (Avicel PH-101) dissolved in a 50 mM sodium phosphate buffer solution (pH 7.0) was added to 100 pL of bacterial cell suspension or 100 pL of culture solution, and incubation was carried out at 30°C. After the incubation, 100 pL of the bacterial cell suspension or 100 pL of the culture solution was separated, and 300 pL of a 1% 3,5-dinitrosalicylic acid solution (10 g/L 3,5-dinitrosalicylic acid, 10 g/L sodium hydroxide, 2 g/L phenol, and 0.5 g/L sodium hydrogen sulfate) was added. After the addition, the bacterial cell suspension or the culture solution was heated at 100°C for 15 minutes to stop the reaction. After the reaction had been stopped, the bacterial cell suspension or the culture solution to which 300 pL of a 40% potassium sodium tartrate aqueous solution had been added was centrifuged to collect a supernatant. For the collected supernatant, the amount of glucose produced was determined by measuring the absorbance at a wavelength of 540 nm. The enzyme activity was calculated from the amount of glucose produced.

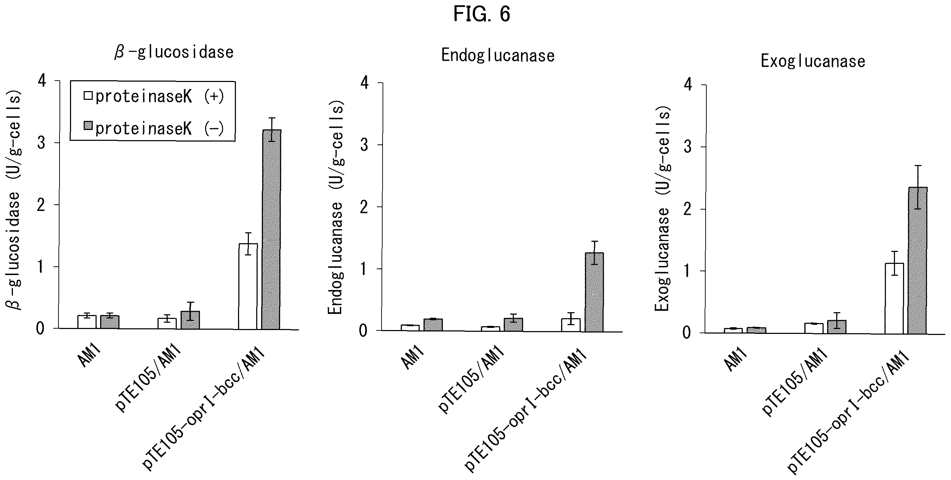

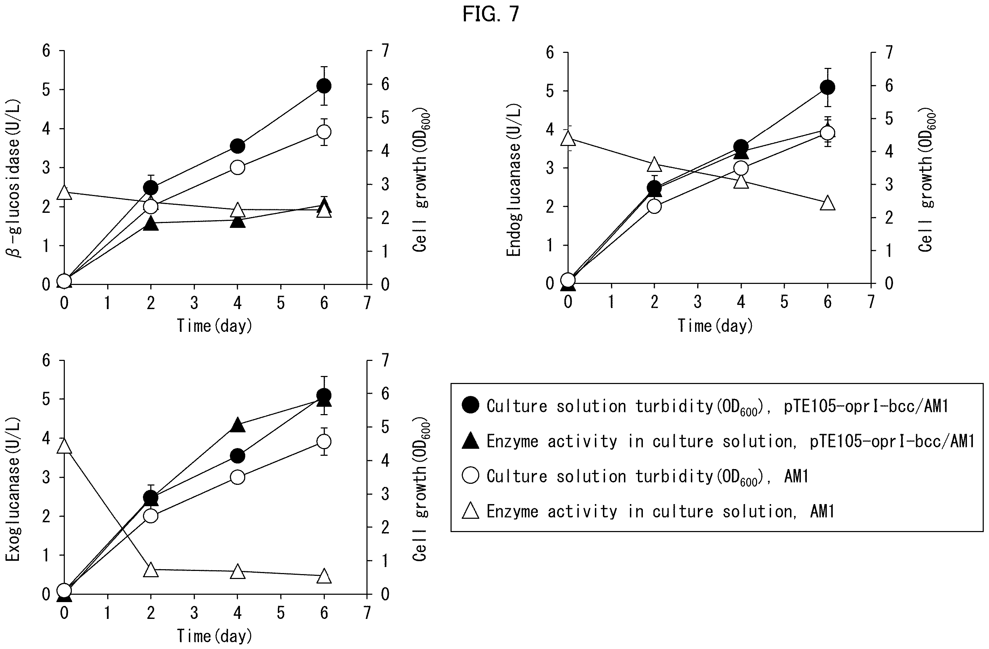

Fig. 6 shows the results of the cellulase activity measurements of the wild strain and the transformants. In Fig. 6, for each enzyme activity, the amount of enzyme for producing a product in an amount of 1 pmol per minute of the reaction time was defined as 1 U. Further, Fig. 7 shows the results of measurement of the change over time of the cellulase activity of the wild strain and *M*. *extorquens* AM1 (pTE 105-oprI-bcc).

In Fig. 6, the culture solution into which *M*. *extorquens* AM 1 (pTE 105-oprI-bcc) that expressed cellulase was inoculated exhibited a clear activity for each of the cellulases. Further, since the cellulase activity of the culture solution was decreased in a case where the proteinase K had been added, it is clear that *M*. *extorquens* AM 1 (pTE 105-oprI-bcc) has each cellulase expressed on a bacterial cell surface thereof.

Further, in Fig. 7, the cellulase activity of the wild strain in the culture solution decreases with the passage of time. This decrease is considered to be caused by protease that is produced by the wild strain. In contrast, it was shown that the cellulase activity of *M*. *extorquens* AM1 (pTE 105-oprI-bcc) in the culture solution is maintained. Thus, it was shown that the use of *M*. *extorquens* AM1 (pTE105-oprI-bcc) enables the cellulase activity in the culture solution to be maintained and enables efficient production of glucose.

### [Examples 3: Synthesis of glucose with use of substrate that is utilizable as raw material]

### <3-1. Production of glucose from substrate other than methanol>

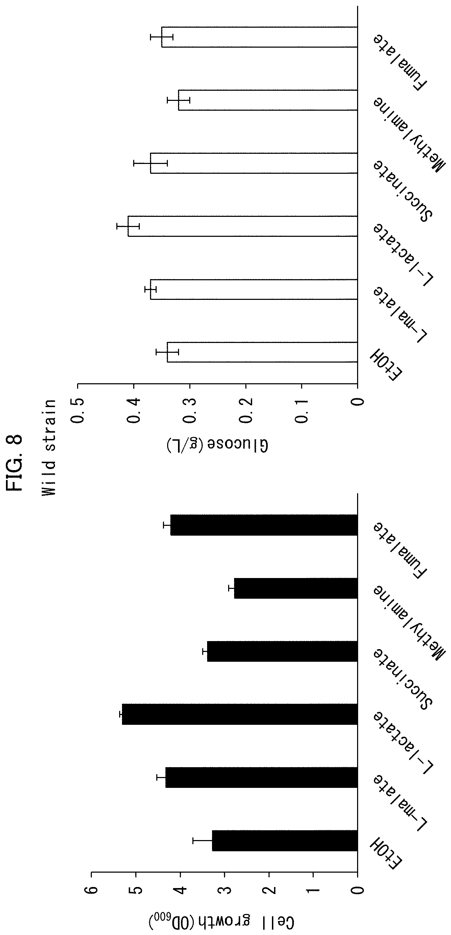

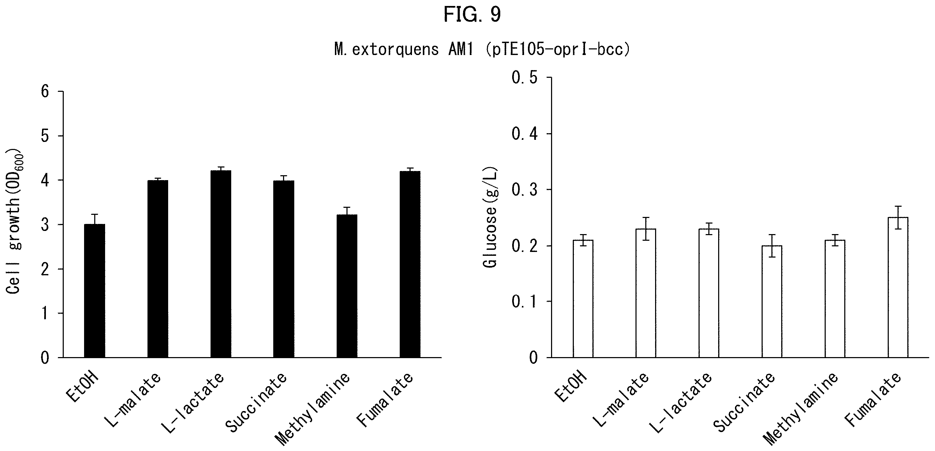

*M. extorquens* AM1 (pTE 105-oprI-bcc) was inoculated into 50 mL of MP medium to which 1 mass% of sodium L-malate, sodium L-lactate, disodium succinate, methylamine hydrochloride, or disodium fumarate or 1 vol% of ethanol, as a substrate, and 20 pg/mL tetracycline had been added. Further, *M. extorquens* AM1 (wild strain) was inoculated into 50 mL of MP medium to which 1 mass% of sodium L-malate, sodium L-lactate, disodium succinate, methylamine hydrochloride, or disodium fumarate or 1 vol% of ethanol, 0.3 U/mL β-glucosidase (Toyobo), and 2 U/mL cellulase (Nacalai Tesque) had been added. The medium into which *M*. *extorquens* AM 1 (pTE 105-oprI-bcc) had been inoculated and the medium into which *M*. *extorquens* AM 1 (wild strain) had been inoculated were each pre-cultured at 30°C at a shake speed of 150 rpm for 5 days. After the bacterial cells had been collected from a pre-culture solution obtained by the pre-culturing, the bacterial cells were washed three times with use of physiological saline and then suspended in 50 mL of physiological saline. The bacterial cell suspension was inoculated into a medium having the same composition as 50 mL of the medium used in the pre-culturing so as to be become 1 vol%, and main culturing was carried out at 30°C at a shake speed of 150 rpm for 5 days. The quantification of glucose was carried out in the same manner as in Example 2. The results of the measurements are shown in Figs. 8 and 9.

Fig. 8 shows the result obtained by using the wild strain, and Fig. 9 shows the result obtained by using *M*. *extorquens* AM 1 (pTE 105-oprI-bcc). Note that, for the substrates in Figs. 8 and 9, the words "sodium" and "hydrochloride" are omitted. In Figs. 8 and 9, the graph in which the vertical axis indicates Cell Growth (OD₆₀₀) shows the result of the measurement of the culture solution turbidity, and the graph in which the vertical axis indicates Glucose shows the result of the quantification of glucose. In Figs. 8 and 9, the culture solution turbidity and the glucose concentration of both the wild strain and *M*. *extorquens* AM1 (pTE 105-oprI-bcc) were sufficiently high whichever substrate was used. Thus, it is clear that both the wild strain and *M*. *extorquens* AM1 (pTE105-oprI-bcc) are capable of producing glucose from a substrate other than methanol.

### [Example 4. Synthesis of Cellobiose with use of substrate that is utilizable as raw material]

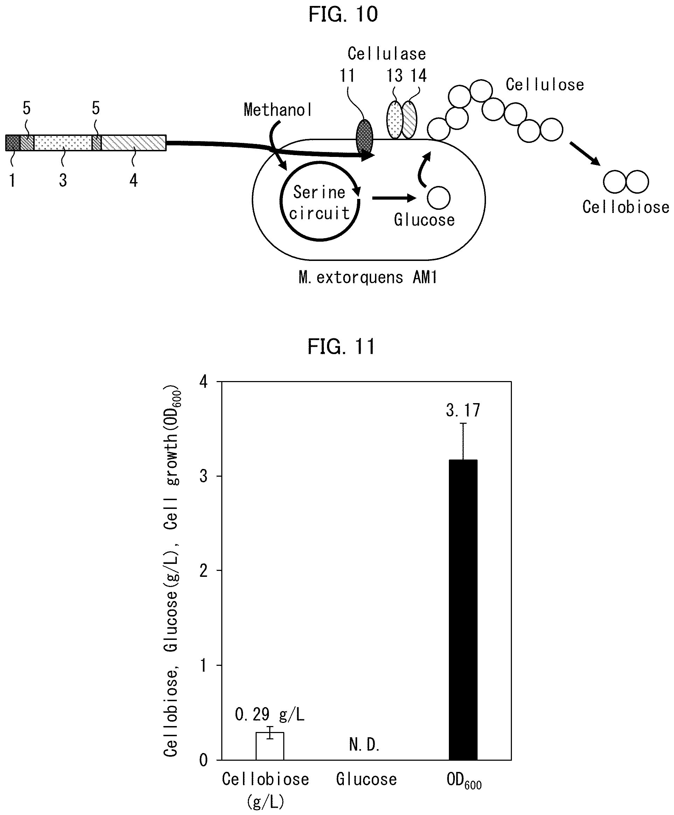

Fig. 10 is a schematic diagram of a transformant prepared in Example 4. As shown in Fig. 10, in Example 4, only the β-glucosidase gene among the genes introduced into the transformant in Example 2 was not introduced. As a result, instead of glucose, cellobiose is produced by the transformant in Example 4.

### <4-1. Construction of plasmid>

### (pTE 105-oprI-cex-cenA)

With use of pTE 105-oprI-bcc as a template and with use of cex-F2 (SEQ ID NO: 27) and oprI-R2 (SEQ ID NO: 26) as primers, pTE 105-oprI-cex-cenA was amplified by PCR. The PCR was carried out under the following conditions: 95°C for 10 seconds; 35 cycles of (95°C for 10 seconds, 70°C for 30 seconds, and 72°C for 4 minutes); and 72°C for 2 minutes. This PCR amplified fragment was purified by gel extraction and mixed with NEBuilder HiFi DNA Assembly Master Mix (NEB) in equal amounts. A resulting mixture was incubated at 55°C for 15 minutes. With use of 5 pL of a resulting reaction product, E. *coli* DH5α was transformed by a heat shock method. After the transformation had been finished, the transformed E. *coli* DH5α was cultured in an LB agar medium (20 pg/mL tetracycline added) to obtain a transformant having a sequence of oprI-cex-cenA (SEQ ID NO: 9). The plasmid was extracted from the transformant with use of MagExtractor -Plasmid- (Toyobo), and it was confirmed by electrophoresis that the extracted plasmid was a target plasmid pTE105-oprI-cex-cenA (hereinafter also referred to as pTE105-oprI-cc).

### <4-2. Method for preparing genetically modified organism>

*M. extorquens* AM1 (JCM 2805, available from Riken BioResource Research Center) was transformed with use of the plasmid which had been constructed in <4-1. Construction of plasmid>. The transformation was carried out by an electroporation method (FEMS microbiol. Lett. 166(1): 1-7 (1998)). After the transformation had been finished, culturing was carried out in a 1 vol% methanol-containing MP agar medium (20 pg/mL tetracycline added) to obtain a transformant *M*. *extorquens* AM1 (pTE105-oprI-cc). Note that the composition of the 1 vol% methanol-containing MP medium is identical to the 1 vol% methanol-containing MP medium used in < 1-2. Preparation of genetically modified organism> .

### <4-3. Cellobiose production from methanol>

*M. extorquens* AM 1 (pTE105-oprI-cc) was inoculated into 50 mL of a 1 vol% methanol-containing MP medium (20 pg/mL tetracycline added), and pre-culturing was carried out at 30°C at a shake speed of 150 rpm for 5 days. After the bacterial cells had been collected from a pre-culture solution obtained by the pre-culturing, the bacterial cells were washed three times with use of physiological saline. The bacterial cells were inoculated into 50 mL of the MP medium such that the culture solution turbidity (OD₆₀₀) at 600 nm became 0.1, and main culturing was carried out at 30°C at a shake speed of 150 rpm for 5 days.

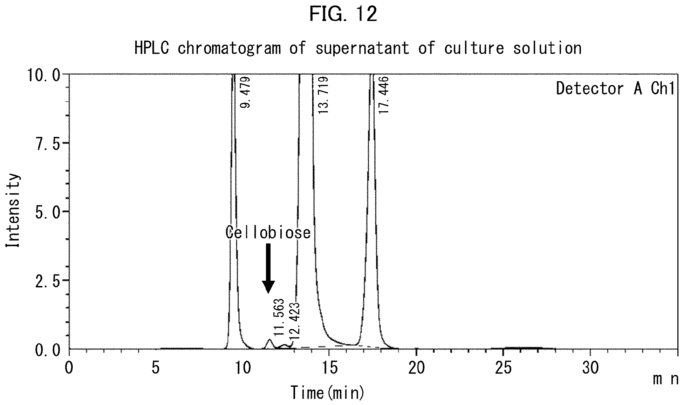

A resulting main culture solution was centrifuged. After an obtained supernatant had been subjected to filtration with a filter (pore diameter: 0.45 pm), a cellobiose yield was measured with use of Prominence (Shimadzu Corporation), which is an HPLC system. A column was Aminex HPX-87H (Bio-Rad), a mobile phase was 5 mM sulfuric acid, a flow rate was 0.4 mL/min, and a column oven temperature was 65°C, and an RI detector was used. The results of the measurements are shown in Figs. 11 and 12.

Fig. 11 indicates that, in a case where pTE 105-oprI-cc was introduced, glucose was not detected from the culture solution, but cellobiose was detected therefrom. Similarly, in Fig. 12, it was shown that cellobiose exists in the culture solution. Therefore, it was shown that the transformant in accordance with an embodiment of the present invention is capable of producing not only glucose, but also cellobiose.

### Industrial Applicability

The present invention can be suitably utilized to produce cellobiose and glucose.

### Reference Signs List

1: oprI gene
2: β-glucosidase gene
3: exoglucanase gene
4: endoglucanase gene
5: linker
11: oprI protein
12: β-glucosidase
13: exoglucanase
14: endoglucanase
20: GFP gene
21: GFP

## Claims

1. A transformant obtained by introducing a cellulase gene and a cell surface protein gene into a cellulose-synthesizing microorganism.

2. The transformant according to claim 1, wherein the cellulose-synthesizing microorganism is a cellulose-synthesizing bacterium.

3. The transformant according to claim 2, wherein the cellulose-synthesizing bacterium is a bacterium belonging to the family *Methylobacteriaceae.*

4. The transformant according to claim 3, wherein the bacterium belonging to the family *Methylobacteriaceae* is a bacterium belonging to the genus *Methylorubrum.*

5. The transformant according to claim 4, wherein the bacterium belonging to the genus *Methylorubrum* is *Methylorubrum extorquens.*

6. The transformant according to claim 1, wherein the cell surface protein gene is an oprI gene.

7. A transformant obtained by introducing an oprI gene into a bacterium belonging to the family *Methylobacteriaceae.*

8. The transformant according to claim 7, wherein the bacterium belonging to the family *Methylobacteriaceae* is a bacterium belonging to the genus *Methylorubrum.*

9. The transformant according to claim 8, wherein the bacterium belonging to the genus *Methylorubrum* is *Methylorubrum extorquens.*

10. The transformant according to claim 6 or 7, wherein the oprI gene is any DNA selected from the group consisting of the following (1) to (6):
(1) DNA consisting of a base sequence of SEQ ID NO: 1;
(2) DNA encoding a polypeptide that has not less than 90% homology to the base sequence of SEQ ID NO: 1 and that is a cell surface protein;
(3) DNA (i) hybridizing, under a stringent condition, with a gene consisting of a base sequence complementary to the base sequence of SEQ ID NO: 1 and (ii) encoding a polypeptide that is a cell surface protein;
(4) DNA encoding a polypeptide consisting of an amino acid sequence of SEQ ID NO: 2;
(5) DNA encoding a polypeptide that has not less than 90% identity to the amino acid sequence of SEQ ID NO: 2 and that is a cell surface protein; and
(6) DNA encoding a polypeptide (i) consisting of an amino acid sequence in which at least one amino acid is deleted, substituted, inserted, or added in the amino acid sequence of SEQ ID NO: 2 and (ii) being a cell surface protein.

11. The transformant according to claim 7, obtained by further introducing a cellulase gene.

12. The transformant according to claim 1 or 11, wherein at least one selected from an endoglucanase gene and an exoglucanase gene is contained as the cellulase gene.

13. The transformant according to claim 12, wherein the exoglucanase gene is at least one DNA selected from the group consisting of the following (1) to (6):
(1) DNA consisting of a base sequence of SEQ ID NO: 5;
(2) DNA encoding a protein that has not less than 90% homology to the base sequence of SEQ ID NO: 5 and that has exoglucanase activity;
(3) DNA (i) hybridizing, under a stringent condition, with a gene consisting of the base sequence of SEQ ID NO: 5 and (ii) encoding a protein that has exoglucanase activity;
(4) DNA encoding a polypeptide consisting of an amino acid sequence of SEQ ID NO: 11;
(5) DNA encoding a protein that has not less than 90% identity to the amino acid sequence of SEQ ID NO: 11 and that has exoglucanase activity; and
(6) DNA encoding a protein (i) consisting of an amino acid sequence in which at least one amino acid is deleted, substituted, inserted, or added in the amino acid sequence of SEQ ID NO: 11 and (ii) having exoglucanase activity.

14. The transformant according to claim 12, wherein the endoglucanase gene is at least one DNA selected from the group consisting of the following (1) to (6):
(1) DNA consisting of a base sequence of SEQ ID NO: 6;
(2) DNA encoding a protein that has not less than 90% homology to the base sequence of SEQ ID NO: 6 and that has endoglucanase activity;
(3) DNA (i) hybridizing, under a stringent condition, with a gene consisting of the base sequence of SEQ ID NO: 6 and (ii) encoding a protein that has endoglucanase activity;
(4) DNA encoding a polypeptide consisting of an amino acid sequence of SEQ ID NO: 12;
(5) DNA encoding a protein that has not less than 90% identity to the amino acid sequence of SEQ ID NO: 12 and that has endoglucanase activity; and
(6) DNA encoding a protein (i) consisting of an amino acid sequence in which at least one amino acid is deleted, substituted, inserted, or added in the amino acid sequence of SEQ ID NO: 12 and (ii) having endoglucanase activity.

15. The transformant according to claim 12, wherein a β-glucosidase gene is further contained as the cellulase gene.

16. The transformant according to claim 15, wherein the β-glucosidase gene is at least one DNA selected from the group consisting of the following (1) to (6):
(1) DNA consisting of a base sequence of SEQ ID NO: 4;
(2) DNA encoding a protein that has not less than 90% homology to the base sequence of SEQ ID NO: 4 and that has β-glucosidase activity;
(3) DNA (i) hybridizing, under a stringent condition, with a gene consisting of the base sequence of SEQ ID NO: 4 and (ii) encoding a protein that has β-glucosidase activity;
(4) DNA encoding a polypeptide consisting of an amino acid sequence of SEQ ID NO: 10;
(5) DNA encoding a protein that has not less than 90% identity to the amino acid sequence of SEQ ID NO: 10 and that has β-glucosidase activity; and
(6) DNA encoding a protein (i) consisting of an amino acid sequence in which at least one amino acid is deleted, substituted, inserted, or added in the amino acid sequence of SEQ ID NO: 10 and (ii) having β-glucosidase activity.

17. A method for producing glucose or cellobiose, comprising a culture step of culturing a transformant according to claim 1 or 11 with use of a carbon source that is utilizable by the transformant.

18. A method for producing an article, comprising a step of producing glucose or cellobiose by the method according to claim 17.

19. A composition comprising glucose or cellobiose obtained by the method according to claim 17.
